# EUROPÄISCHE PATENTANMELDUNG

(11) **EP 0 766 964 A1**
(43) Veröffentlichungstag der Anmeldung: **09.04.1997**
(21) Anmeldenummer: 96112847.7
(22) Anmeldetag: 09.08.1996
(51) Int. Cl.: A61K 31/505

(54) **Verwendung von Orotsäure zur Herstellung eines Arzneimittels zur schützenden Behandlung von Membranen und des Endothels**

(30) Priorität: 17.08.1995 DE 19530300
(71) Anmelder: WÖRWAG PHARMA GmbH, 71034 Böblingen (DE)
(72) Erfinder: Wörwag, Fritz, Dr., 70192 Stuttgart (DE)
(74) Vertreter: Weller, Wolfgang, Dr.rer.nat.

(57) **Zusammenfassung**

Verwendung von Orotsäure zur Verbesserung oder zum Erhalt der Integrität von Körperzellen, insbesondere zum Schutz von Membranen und des Endothels.

## Beschreibung

Die vorliegende Erfindung betrifft ein Produkt für die Verbesserung oder den Erhalt der Integrität von Körperzellen, insbesondere den prophylaktischen und therapeutischen Schutz von Membranen und des Endothels.

Es ist bekannt, daß das Endothel, die einschichtige zellige innere Schutzschicht der Gefäße, und allgemein die Membranen von Zellen durch Ischämie, durch mechanische Belastungen, durch Beanspruchung etc. geschädigt werden.

Die Zellmembranen, deren Funktion vor allem darin besteht, die Inhaltsstoffe und Organellen der Zelle einzuschließen, werden z.B. durch chemische Verbindungen (z. B. Umweltnoxen, "Freie Radikale", Cholesterin), physikalische Einflüsse (z. B. UV-Strahlung, Radioaktivität) oder durch starke körperliche Belastungen geschädigt. Diese Belastungen können im Zusammenhang mit Sport, Unfällen etc. auftreten.

Auch eine Ischämie, also eine Minderdurchblutung infolge von z.B. Herzinfarkt oder Arteriosklerose, kann zu Schädigungen der Membranen führen.

Diese Schädigungen führen dazu, daß die Membranen ihre Festigkeit verlieren und ihre Funktion des Zellschutzes, der Gewährleistung der Spezifität der Zelle, nicht mehr erfüllen können.

Die Schäden an der Membran äußern sich in einem sogenannten "Lecken", die Zelle verliert ihre Inhaltsstoffe und wird umgekehrt mit anderen Stoffen überladen. Die osmotischen Verhältnisse werden gestört, so daß die Zelle Elektrolyte, Magnesium, Kalium, intrazelluläre Enzyme etc. verliert und z.B. Calcium und Natrium aufnimmt. Im Extremfall kann diese Schädigung zum Absterben der Zelle führen.

Bekannte Symptome dieser Schädigungen sind z.B. Muskelverhärtungen sowie Verlust an Muskelgewebe nach starken sportlichen oder sonstigen körperlichen Anstrengungen.

Auch das Endothel ist empfindlich gegen mechanische Verletzungen und Ischämie. Die Schäden können hier z.B. durch Bluthochdruck hervorgerufen werden.

Die Zerstörung oder Schädigung des Endothels bewirkt, daß die Gefäße ihre Elastizität verlieren und sich z.B. arteriosklerotische Plaques bilden, so daß eine Minderversorgung auftreten kann und das Regelvermögen für den Blutdruck zurückgeht.

Die Behandlung der Membranschädigungen erfolgte bisher rein symptomatisch, indem Sportlern z.B. bei Muskelkater oder -verhärtung Salben appliziert oder Muskelrelaxantien oder Anabolika verabreicht wurden bzw. ein Aufbautraining durchgeführt wurde, um zerstörte Muskelmasse wieder aufzubauen.

Bei Ischämie durch Herzinfarkt besteht eine weitere Behandlung in der Verabreichung von Herzmitteln oder letztendlich in einer Bypass-Operation oder Herztransplantation.

Auch bei der Endothel-Schädigung wurden bisher die Folgeerkrankungen bzw. die Symptome behandelt, indem z.B. der Blutdruck durch Medikamente beeinflußt wurde.

Zahlreiche in vitro- und in vivo-Experimente sowie klinische Studien haben gezeigt, daß der anfängliche Schritt der arteriosklerotischen Veränderung die funktionale und/oder morphologische Störung der Membranen sowie der inneren und äußeren Membransysteme der Zellen in den Blutgefäßwänden ist.

Hyperlipidämie und Hypercholesterinämie erhöhen die Permeabilität der Gefäßwand. Eine cholesterinreiche Diät induziert eine intra- und extrazelluläre Lipidansammlung in den Gefäßen, wobei sich manchmal anorganisches Calcium irreversibel in den Läsionen ansammelt. Dies sind die bekanntesten Änderungen in der Aorta und in den Koronarien, sie treten jedoch auch in den peripheren Gefäßen auf.

Vor diesem Hintergrund ist es Aufgabe der vorliegenden Erfindung, ein Produkt zur Verbesserung oder zum Erhalt der Integrität von Körperzellen, insbesondere zum Schutz von Membranen und des Endothels zur Verfügung zu stellen.

Erfindungsgemäß wird diese Aufgabe dadurch gelöst, daß Orotsäure zur Verbesserung oder zum Erhalt der Integrität von Körperzellen, insbesondere zum Schutz von Membranen und des Endothels verwendet wird.

Es wurde nämlich überraschenderweise gefunden, daß Orotsäure präventiv und therapeutisch zur schützenden Behandlung von Membranen und des Endothels eingesetzt werden kann, da sie die Integrität der Körperzellen verbessert oder erhält.

Eine Ursache dafür könnte sein, daß Orotsäure, die eine Intermediärsubstanz der Pyrimidinsynthese ist, auch die Synthese von Membranbausteinen induzieren könnte.

Orotsäure wird bisher in Salzform als sogenannter Mineral- oder Elektrolytschlepper eingesetzt und dient dazu, bestimmte Kationen in die Zelle zu transportieren. Aus der DE-OS-24 10 181 ist es beispielsweise bekannt, Lithiumorotat zu verwenden, um Natrium, das bei der Regulierung des Wasserhaushaltes eine Rolle spielt, in der Zelle durch Lithium zu ersetzen. Lithium wird in Form des Salzes der Orotsäure verwendet, da dieses den Transport des Lithiums in die Zelle bewirken kann.

Durch die Deplazierung des Natriums durch Lithium wird die Menge an gespeichertem Wasser reduziert. Dieser Effekt wird zur Therapie von Hypertonie, Arteriosklerose, Gefäß- und Gewebserkrankungen insbesondere im zerebralen Bereich sowie zur Dauer- und Schutzbehandlung von Migräne ausgenutzt.

Überraschenderweise wurde jetzt gefunden, daß die Orotsäure selbst präventiv und therapeutisch wirkt und als Schutz-Mittel für Membranen und das Endothel eingesetzt werden kann.

Die Orotsäure kann dabei direkt oder in Form eines ihrer Salze verwendet werden, wobei vorzugsweise Magnesiumorotat eingesetzt wird.

Bei Tierexperimenten mit Wistar Ratten wurden bewußt Schädigungen am Endothel und an den Membranen durchgeführt, und zwar einerseits durch kurzfristige Hypoxämie und durch langzeitige maligne Hypertonie. In der nichtbehandelten Gruppe wurden nach histologischer Bearbeitung von verschiedenen Arterien und der Herzmuskulatur Gefäßintimaveränderungen, pathologische Strukturen und Funktionserscheinungen, erhöhte Permeabilität, Störungen des Calciumhaushalts und mitochondriale Funktionsstörungen festgestellt. In einer mit Magnesiumorotat behandelten Gruppe wurde festgestellt, daß die Gefäß- und Herzmuskulaturintegrität gut erhalten geblieben ist, und daß weniger pathologische Veränderungen stattgefunden haben.

Daraus konnte geschlossen werden, daß die Orotsäure eine endothel- und membranschützende Wirkung ausübt.

Untersuchungen an Triathleten haben nämlich gezeigt, daß nach einer Magnesiumorotat-Supplementierung der Membrangradient aufrechterhalten werden kann, was ein Anzeichen dafür ist, daß die Membranintegrität trotz der starken körperlichen Belastungen während des Triathlons aufrechterhalten bleibt.

Ferner konnte in Tierexperimenten gezeigt werden, daß Orotsäure arteriosklerotischen Gefäßveränderungen unter experimentellem Cholesterinstreß entgegenwirkt. Dabei zeigte sich, daß die Wirkung der reinen Orotsäure deutlich besser war als die von MgCl₂, so daß der Wirkeffekt bei Magnesiumorotat nicht primär auf das Magnesium, sondern auf die Orotsäure zurückzuführen ist.

Weiterhin wurde in einem Tierexperiment gezeigt, daß Orotsäure blutdrucksenkend wirkt.

Die blutdrucksenkende Wirkung sowie die Verhinderung der Arteriosklerose wirken sich nun positiv auf den Schutz des Endothels aus, da durch die Orotsäure bzw. durch Magnesiumorotat der Blutdruck gesenkt werden kann, so daß die mit hohem Blutdruck verbundenen Schädigungen des Endothels vermieden werden. Auch die Verhinderung oder Zurückführung der Arteriosklerose durch diese Präparate deutet an, daß das Endothel präventiv geschützt und therapeutisch behandelt werden kann.

Aus all dem ergibt sich, daß Orotsäure und Magnesiumorotat dazu eingesetzt werden können, um die Integrität von Körperzellen zu verbessern oder zu erhalten.

Dieser Effekt ist als überraschend anzusehen; er war in dieser Deutlichkeit und Ausprägung nicht zu erwarten.

In einem Ausführungsbeispiel ist es dann bevorzugt, wenn eine Mischung aus Orotsäure und einer Magnesiumverbindung verwendet wird.

Hier ist von Vorteil, daß ein synergistischer Effekt auftritt, zum einen wird die Wirkung der Orotsäure ausgenutzt, wobei zusätzlich eine Magnesiumverbindung eingesetzt wird, um auch deren Wirkung mit zu verwenden.

Die Mischung besteht dabei vorzugsweise aus einem Salz der Orotsäure und einer Magnesiumverbindung, wobei auch eine Mischung aus Orotsäure und Magnesiumorotat verwendet werden kann.

Auch hier ist von Vorteil, daß neben der reinen Orotsäure Magnesium in dem herzustellenden Arzneimittel/Präparat/Produkt zu finden ist.

Das aus Orotsäure und/oder ihren Salzen hergestellte Arzneimittel/Produkt kann dabei in allen Darreichungsformen auftreten.

Die Erfindung wird nachfolgend anhand einiger Studien und im Zusammenhang mit den Figuren näher beschrieben und erläutert.

Es zeigen:
- Fig. 1: Indizes arteriosklerotischer Gefäßveränderungen unter experimentellem "Cholesterinstreß" bei Kaninchen; und
- Fig. 2: die Reduktion der in Wistar-Ratten experimentell induzierten, malignen Blutdrucksteigerung durch Magnesiumorotat.

### Beispiel 1: Untersuchung der Wirkung von Orotsäure und Magnesiumorotat in einem Arteriosklerose-Modell

In einer Versuchsreihe mit einem Arteriosklerose-Modell wurden Kaninchen über 112 Tage einer zweiprozentigen Cholesterindiät ausgesetzt und mit äquimolaren Mengen von Magnesiumchlorid, Orotsäure und Magnesiumorotat bzw. in der Kontrollgruppe überhaupt nicht weiter behandelt. Zusätzlich zu histologischen Untersuchungen der Koronaarterien sowie der Aorta femoralis und renalis wurde der Zustand der Aorta an jeweils mehreren Stellen auch planimetrisch-photographisch dokumentiert. Mittels einer computergestützten, digitalen Methode der Oberflächenmessung ("density slice"-Methode) erhält man hierbei dreidimensionale Bilder, mit deren Hilfe auch eine Quantifizierung der cholesterininduzierten Lumeneinengungen möglich ist.

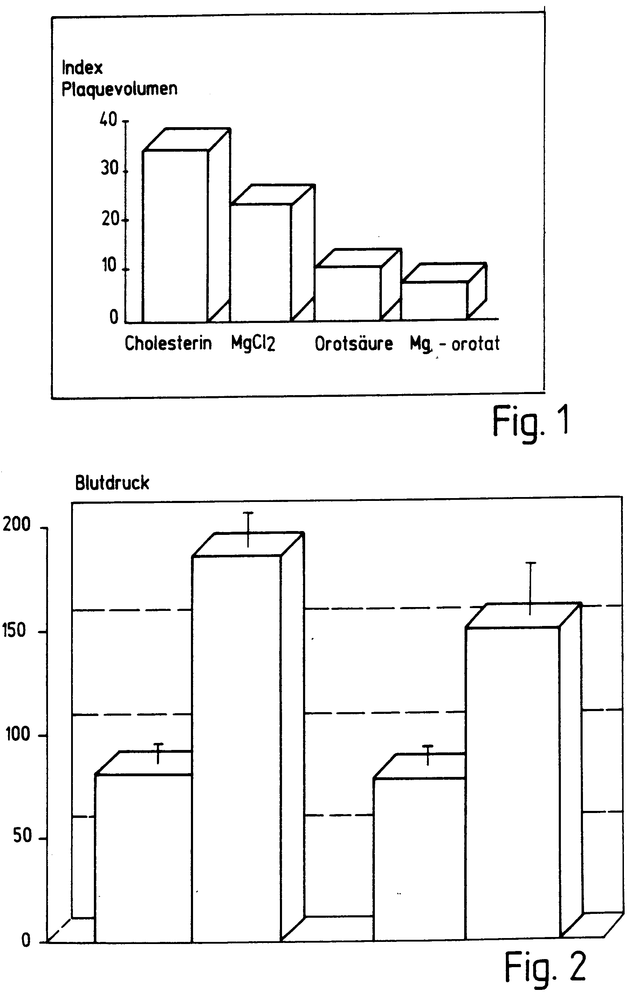

In Fig. 1 ist das Ergebnis dieser Untersuchung dargestellt. Es ist zu erkennen, daß die Gefäßverengungen in der unbehandelten Kontrollgruppe mit einem Index von 34,36 am ausgeprägtesten waren. Die Orotsäure wirkt mit einem Index von 9,77 deutlich besser den arteriosklerotischen Läsionen entgegen als Magnesiumchlorid (Index 22,63).

Als bester Schutz erwies sich jedoch Magnesiumorotat mit einem Index von 7,28.

Diese quantifizierten Ergebnisse wurden durch die licht- bzw. elektronenmikroskopischen Befunde sowie durch enzymhistochemische Untersuchungen bestätigt. Demnach verhütet Magnesiumorotat auch in den Koronarien die Genese sklerotischer Läsionen am besten. Die lumeneinengende, durch die Cholesterinfütterung induzierte Proliferation von Schaumzellen war unter dieser Medikation weitaus am geringsten ausgeprägt.

Aus dieser Untersuchung ergibt sich, daß Orotsäure selbst arteriosklerotische Gefäßveränderungen entscheidend verhütet und daß diese Wirkung durch Zugabe von Magnesium weiter gesteigert werden kann.

### Beispiel 2: Untersuchung der Wirkung von Magnesiumorotat im Hypertonie-Tiermodell

In den Hypertonie-Tiermodellen nach Rojo-Ortega-Genest (ROG-Modell) und Lörincz-Goracz (LG-Modell) wird bei Wistar-Ratten die Aorta zwischen den beiden Renalarterien unterbunden bzw. die Niere in eine Gummimanschette fest eingepreßt, was einen malignen Hypertonus hervorruft, also eine Hypertonie mit konstanter Erhöhung des arteriellen diastolischen Blutdruckes auf > 120 mmHg.

Unter täglicher Zufuhr von 300 mg Magnesiumorotat waren weit weniger periarterielle Zellinfiltrationen und Herzmuskelzellausfälle zu beobachten als ohne Behandlung.

Das Ergebnis dieser Untersuchung ist in Fig. 2 dargestellt, wobei die dunklen Säulen jeweils den Beginn der ROG-Hypertonie anzeigen, während die hellen Säulen das Ende der ROG-Hypertonie darstellen. Links in Fig. 2 ist die ROG-Hypertonie ohne Behandlung gezeigt, während die beiden rechten Säulen den Verlauf der ROG-Hypertonie unter Zugabe von täglich 300 mg Magnesiumorotat wiedergibt.

Diese Therapie reduzierte die experimentiell induzierte, maligne Blutdrucksteigerung per se. Der Wirkeffekt des Magnesiumorotats ergibt sich dabei aus der Differenz zwischen den beiden hellen Säulen. Der Blutdruck betrug im ROG-Modell 152 mmHg systolisch gegenüber 186 mmHg bei unbehandelten Kontrolltieren nach Ausgangswerten von 79 bzw. 81 mmHg, war also bei den behandelten Tieren deutlich geringer ausgeprägt.

### Beispiel 3: Untersuchung der Wirkung von Magnesiumorotat im Hypoxämie-Tiermodell

In dem Hypoxämie-Tiermodell nach Rojo-Ortega-Gentest und nach Lorincz-Goracz wurde eine kurzfristige Hypoxämie, also eine Herabsetzung des Sauerstoffgehaltes im Blut, bewerkstelligt. In der nichtbehandelten Gruppe wurde eine histologische Bearbeitung verschiedener Arterien und der Herzmuskulator durchgeführt und Gefäßintimaveränderungen, eine pathologische Struktur, eine erhöhte Permeabilität, Störungen des Calciumhaushaltes und mitochondriale Funktionsstörungen festgestellt. Bei mit 200 mg pro kg und Tag mit Magnesiumorotat therapierten Ratten wurde bei den entsprechenden histologischen Untersuchungen festgestellt, daß die Gefäß- und Herzmuskulaturintegrität erhalten geblieben ist, und daß kaum pathologische Veränderungen stattgefunden haben.

### Beispiel 4: Untersuchung der Wirkung von Magnesiumorotat auf den Membranstoffwechsel von Triathleten

In einer doppelblinden, randomisierten und placebokontrollierten Studie wurden 23 Triathleten einem 500 m Schwimm-, 20 km Fahrrad- und 5.000 m Lauftest nach einer vierwöchigen Magnesiumorotat-Supplementierung von 17 mmol/Tag unterworfen. Die Tests wurden ohne Pausen direkt hintereinander absolviert. Vor dem Test, zwischen den Disziplinen und am Ende wurde den Probanden Blut entnommen und auf Parameter des Membranstoffwechsels untersucht.

Zur Untersuchung der Membran wurde jetzt die Konzentration von Creatinkinase im Blut untersucht. Creatinkinase ist eines der Enzyme, das die Zelle bei Schädigung der Membran verliert, so daß es für die Messung der Membranintegrität als Leitenzym verwendet werden kann. Mit anderen Worten, die Anwesenheit von Creatinkinase und Creatinin im Blut deutet auf Membranschädigungen hin.

In dieser Untersuchung lagen nun die streßbedingten Creatininkonzentrationen in der Verumgruppe durchweg niedriger als die der Kontrollgruppe. Die streßbedingte, relative Erhöhung der Creatininkonzentration war in der Verumgruppe mit 124 ± 18 % im Vergleich zur Placebogruppe mit 138 ± 15 % mit p = 0,07 fast statistisch signifikant niedriger.

Der relative, streßbedingte Anstieg der Creatinkinase-Aktivität im Serum fiel in der Kontrollgruppe mit 41 % signifikant höher aus als in der Verumgruppe mit 22 %.

Die verminderte streßbedingte Freisetzung der Creatinkinase durch Magnesiumorotat-Supplementierung deutet auf eine verbesserte Aufrechterhaltung der Membrangradienten während des Triathlons in der Verumgruppe hin.

### Beispiel 5: Auswirkung der Langzeitbehandlung mit Orotsäure

An zehn Patienten, die langjährig mit Orotsäure behandelt wurden, konnten nach sonographischer Kontrolle der Karotiden, also der paarigen gemeinsamen Kopfarterien im Halsbereich, überraschenderweise keine wesentlichen pathologischen Befunde erhoben werden. Dies ist auf einen wirksamen Edothelschutz durch die Verabreichung der Orotsäure zurückzuführen.

Die obigen Beispiele zeigen, daß Orotsäure für sich genommen und insbesondere Magnesiumorotat den Membrangradienten aufrechterhält, arteriosklerotischen Gefäßänderungen entgegenwirkt und blutdrucksenkend wirkt. Aus all diesem ergibt sich, daß die Orotsäure bzw. das Magnesiumorotat nicht nur die Zellmembranen sondern auch das Endothel schützen.

## Patentansprüche

1. Verwendung von Orotsäure zur Herstellung eines Arzneimittels zur schützenden prophylaktischen oder therapeutischen Behandlung der Integrität von Körperzellen, insbesondere von Membranen und des Endothels.

2. Verwendung nach Anspruch 1, dadurch gekennzeichnet, daß ein Salz der Orotsäure verwendet wird.

3. Verwendung nach Anspruch 2, dadurch gekennzeichnet, daß Magnesiumorotat verwendet wird.

4. Verwendung nach einem der Ansprüche 1 - 3, dadurch gekennzeichnet, daß eine Mischung aus Orotsäure und einer Magnesiumverbindung verwendet wird.

5. Verwendung nach Anspruch 4, dadurch gekennzeichnet, daß eine Mischung aus einem Salz der Orotsäure und einer Magnesiumverbindung verwendet wird.

6. Verwendung nach Anspruch 4 oder Anspruch 5, dadurch gekennzeichnet, daß eine Mischung aus Orotsäure und Magnesiumorotat verwendet wird.
